# EUROPÄISCHE PATENTSCHRIFT

(11) **EP 1 223 159 B1**
(45) Veröffentlichungstag und Bekanntmachung des Hinweises auf die Patenterteilung: **15.12.2004**
(21) Anmeldenummer: 01130366.6
(22) Anmeldetag: 20.12.2001
(51) Int. Cl.: C07C 67/08, C07C 69/54, G02B 1/04, A61K 6/02

(54) **Herstellung unsymmetrischer (Meth)acrylatvernetzer**
Preparation of asymmetrical (meth)acrylate crosslinking agents
Préparation d'agents de réticulation asymétriques à base de (meth)acrylate

(30) Priorität: 13.01.2001 DE 10101389
(43) Veröffentlichungstag der Anmeldung: 17.07.2002
(73) Patentinhaber: Röhm GmbH & Co. KG, 64293 Darmstadt (DE)
(72) Erfinder: Siol, Werner, Dr., 64297 Darmstadt (DE)

(56) Entgegenhaltungen:
- EP-A- 0 899 286
- SAIMI YASUKAZU ET AL: "Preparation and visible light polymerization of triethyleneglycol acrylate methacrylate" POLYMER JOURNAL, Bd. 24, Nr. 4, 1992, Seiten 357-363, XP009019095 ISSN: 0032-3896
- PATENT ABSTRACTS OF JAPAN vol. 013, no. 293 (C-615), 6. Juli 1989 (1989-07-06) & JP 01 087608 A (MITSUI PETROCHEM IND LTD), 31. März 1989 (1989-03-31)
- PATENT ABSTRACTS OF JAPAN vol. 007, no. 108 (C-165), 11. Mai 1983 (1983-05-11) & JP 58 029744 A (TOUYOU KONTAKUTORENZU KK), 22. Februar 1983 (1983-02-22)
- DATABASE WPI Section Ch, Week 197932 Derwent Publications Ltd., London, GB; Class A41, AN 1979-59492B XP002258093 & SU 630 249 A (AS USSR HIGH MOLECU), 13. September 1978 (1978-09-13)

## Beschreibung

Die Erfindung betrifft ein Verfahren zur Herstellung von unsymmetrischen (Meth)acrylatvernetzern der allgemeinen Formel (I)

(I) CH₂=CHCOO-[-(CH₂)ₓ-CHR-O-]ₙ-COCCH₃=CH₂

mit x = 1, 2, 3, R = H, CH₃, n = 1- 100
sowie die Verwendung der nach diesem Verfahren hergestellten Vernetzer zur Synthese von Superabsorbern und Verdickungsmitteln.

### Stand der Technik

(Meth)acrylsäureester mehrwertiger Alkohole, z.B. Ethylenglykoldimethacrylat, haben breite technische Anwendung gefunden (siehe z.B. Kirk-Othmer, Encyclopedia of Chemical Technology, Vol. 13, 331ff). Die Synthese der verschiedenen Acrylsäure- und Methacrylsäureester erfolgt im allgemeinen durch Veresterung der entsprechenden Alkanole mit Acrylsäure oder Methacrylsäure oder durch Umesterung z.B. Methylmethacrylat oder Acrylaten.
Darüber hinaus werden auch Acrylsäurechlorid bzw. Methacrylsäurechlorid zur Synthese von (Meth)acrylsäurestern eingesetzt. Insbesondere im Falle der Acrylsäureester ist jedoch bei diesem Verfahren auf eine wirksame Bindung der frei werdenden HCl zu achten, um die Bildung von chlorhaltigen Nebenprodukten zu vermeiden .
Auch die Synthese von (Meth)acrylsäureestern mit Hilfe von Anhydriden wird beschrieben. So beschreibt USP 5,491,244 die Synthese eines Epoxygruppen haltigen Acrylsäureesters. Die Synthese erfolgt ohne Katalysator aus dem entsprechenden Epoxyalkohol und einem etwa vierfachen Überschuß an Acrylsäureanhydrid. Deutlich schlechter verläuft die Synthese von Methacrylsäureestern ausgehend von Methacrylsäureanhydrid und verschiedenen Alkanolen mit einer zusätzlichen Esterfunktion (Milchsäureestern). Trotz Katalyse mit Schwefelsäure, einem Überschuß an Methacrylsäureanhydrid und 5 Stunden Erhitzen auf 130°C wird nur ein Umsatz von ca 50% erreicht. Darüber hinaus bereitet die Abtrennung des nicht umgestzten Methacrylsäureanhydrids Probleme (C.E.Rehberg et al., Journal of the American Chemical Society, Vol.67, 210 (1945)).

Ein elegantes Verfahren zum Aufbau von α, ω - di-(meth)acrylsubstituierten Polytetrahydrofuran wird von Heitz und Mitarbeitern berichtet ( USP 4,412,063 ). Dabei wird Tetrahydrofuran in Gegenwart von Acrylsäure- bzw. Methacrylsäureanhydrid unter Bildung von Polytetrahydrofuran mit Acrylat- bzw. Methacrylatendgruppen polymerisiert.

Neben Verbindungen mit gleichartigen Vinylgruppen wie z.B. die oben genannten Diacrylate bzw. Dimethacrylate haben insbesondere auch Divinylverbindungen mit Doppelbindungen unterschiedlicher Reaktivität Interesse gefunden. Hier sind z.B. Methacrylsäurevinyl- oder Methacrylsäureallylester zu nennen. Diese sogenannten Pfropfvernetzer sind zum Beispiel zum Aufbau gut miteinander verknüpfter mehrphasiger Kunststoffe geeignet, da im Verlauf der Polymerisation zunächst nur die Methacryloylgruppe copolymerisiert wird, während die Allylgruppe erst zu einem späteren Zeitpunkt am Polymerisationsprozess teilnimmt. Dies ermöglicht z.B. die Pfropfung von Polybutylacrylat auf ein Allylgruppen enthaltendes Polymethylmethacrylat (siehe z.B. DE-PS 3329765.7).

Von besonderem Interesse sind Vernetzer, die eine Acryloyl- und eine Methacryloylgruppe in einem Molelekül aufweisen. Methacryloyl- und Acryloylgruppe unterscheiden sich hinsichtlich ihres Copolymerisationsverhaltens deutlich. Z.B. findet man für das System Methylacrylat= Monomer 1, Methylmethacrylat= Monomer 2 die Copolymerisationsparameter r₁ = 0,40, r₂= 2,15 (siehe z.B. Ullmann's Encyclopedia of Industrial Chemistry, Fifth Edition, Vol. A21, 162), so dass auch hier in der Anfangsphase der Polymerisation vor allem die Methacryloylgruppe des Vernetzermoleküls in die Kette eingebaut wird. Das Coplymerisationsverhalten der Acryloylgruppe ist jedoch nicht so ungünstig, dass auch diese Gruppe im Verlauf der Polymerisation nahezu vollständig in die Polymerketten eingebaut wird.
Vernetzer mit einer Acryloyl- und Methacryloyl-gruppe in einem Molekül lassen sich daher mit Vorteil zum Aufbau sehr homogener Netzwerke einsetzen.

So wird Methacryolyloxyethylacrylat als Rezepturbestandteil für die Herstellung weicher Kontaktlinsen eingesetzt ( JP 58 28,718 ). Die Wasseraufnahme der so hergestellten Kontaktlinsen beträgt 70,9% verglichen mit nur 29,0% bei herkömmlichen Linsen.

Verbindungen vom Typ CH₂=CHCO₂Z¹Z²Z³O₂ CC(Me)=CH₂ [Z¹ ,Z³ = (CHCHO)ₙ, (n=0-10); Z² = alkyl,aryl,cycloalk] können für härtbare Dentalzemente eingesetzt werden. So zeigt ein Produkt, hergestellt mit Methacryloyloxyethylacrylat eine Druckfestigkeit von 43,600 kg/cm² und eine Brinellhärte von 40,7 gegenüber 33,300 kg/cm² und 27,2 bei Verwendung von Ethylenglykoldimethacrylat (JP 0187,608).

U.S.S.R. 630,249 empfiehlt unsymmetrische (Meth)acrylatvernetzer der Formel

CH₂=CMeCO₂ZO₂CCH=CH₂

[Z = CH₂CH₂, CHMeCH₂, CH₂CH₂OCH₂CH₂ ] zum Aufbau wärmeformbeständiger Polymerer. Die Synthese dieser (Meth)acrylatvernetzer erfolgt durch Umsetzung von CH₂=CMeCO₂ZOH mit CH₂=CHCOCl in Dimethylacetamid.

JP 58 29,744 beschreibt die Synthese von Methacryloyloxyethylacrylat mit einer Reinheit von 99,7% durch Umsetzung von Hydroxethylacrylat mit Methacrylsäurechlorid.Dabei wird unterhalb von 10°C gearbeitet und die entstehende HCl mit Triethylamin gebunden.Das (Meth)acrylatmonomere wird zur Herstellung weicher Kontaktlinsen eingesetzt.

EP 0 899 286 (Toyo Zink Manufacturing Co.) beschreibt vinylgruppenhaltige Dendrimere, die als Bindemittel für Beschichtungen eingesetzt werden. Die Dendrimere ergeben Polymere mit hohen Molekulargewichten und recht geringen Viskositäten. Durch die Verwendung der Dendrimere in den Beschichtungsformulierungen lässt sich der Anteil an niedermolekularen Verbindungen gering halten.

Saimi, lshihara und Nakabayashi (Y. Saimi, K. Ishihara und N. Nekabayashi, Polymer Journal, 24, (1992), Seite 357 - 363) beschreiben die Synthese von Triethylenglycol(meth)acrylat durch Umsetzung von (Meth)acrylsäurechlorid mit Triethylenglycol. Als Fänger für die bei der Reaktion entstehende Salzsäure muss eine basische Verbindung, beispielsweise Triethylamin, eingesetzt werden. Das entstandene Triethylaminhydrochlorid muss mit Wasser aus der Reaktionsmischung ausgewaschen werden, anschließend muss die Reaktionsmischung noch neutralisiert und getrocknet werden. Diese Aufarbeitungsschritte sind umständlich und verringern die Ausbeute.

### Aufgabe

Obwohl unsymmetrisch substituierte (Meth)acrylatvernetzer zum Aufbau verschiedener Polymernetzwerke also von Interesse sind, fehlt es bislang an einem geeigneten Verfahren zur Bereitstellung dieser Monomerklasse. Der im Markt verfügbare Methacrylsäure- [3-acryloxy]- 2-hydroxypropylester, der durch Umsetzung von Glycidylmethacrylat mit Acrylsäure zugänglich ist, stellt zwar einen unsymmetrischen Methacrylatvernetzer dar, aufgrund seiner Hydroxylgruppe und der empfindlichen Glycerinstruktur ist dieser Vernetzer jedoch nur begrenzt einsetzbar. Nach wie vor fehlt es an einem Verfahren, das geeignete unsymmetrische (Meth)acrylatvernetzer bereit-stellt und dabei insbesondere Produkte ohne chlorhaltige Verunreinigungen liefert.

### Lösung

Es wurde nun gefunden, dass unsymmetrische (Meth)acrylatvernetzter der Formel (I)

(I) CH₂=CHCOO-[-(CH₂)ₓ-CHR-O-]ₙ-COCCH₃=CH₂

mit x = 1,2,3, R = H, CH₃, n = 1- 100
in einfacher Weise dadurch zugänglich sind, dass Hydroxyacrylate gemäss Formel (II)

(II) CH₂=CHCOO-[-(CH₂)ₓ-CHR-O-]ₙ-H

mit Methacrylsäureanhydrid unter Bildung von (I) und Methacrylsäure umgesetzt werden.
Die Umsetzung erfolgt im allgemeinen in Gegenwart von 0,1- 5 Gew% eines sauren Katalysators in einem Temperaturbereich von 0- 100°C in einem Zeitraum von 0,5- 24 h. Unter diesen Bedingungen erfolgt die Veresterung von (II) zu (I), die Acrylsäureestergruppe wird jedoch nicht gespalten, so dass das Produkt (I) nur einen geringen Gehalt an Diacrylat und Dimethacrylat enthält. Üblicherweise ist der Gehalt an Diacrylat- und Dimethacrylat < 5 Gew%, bevorzugt < 2 Gew%. Zu einem wesentlichen Anteil resultiert der Gehalt an Diacrylat von bereits im Ausgangsmaterial (II) enthaltenen Diacrylat, während der Gehalt an Dimethacrylat im Endprodukt vor allem auf Verunreinigungen vom Typ (III) im Ausgangsmaterial (II) zurückzuführen ist.

(III) HO-[-(CH₂)ₓ-CHR-O-]ₙ-H

In der Regel ist sowohl der Gehalt an Diacrylat als auch der Gehalt an Dimethacrylat im Produkt < 1 Gew%.
Prinzipiell liefert das erfindungsgemässe Verfahren unsymmetrische (Meth)acrylatvernetzer (I) frei von chlorhaltigen Nebenprodukten. Im allgemeinen ist ein Gehalt an chlorhaltigen Verunreinigungen < 0,1Gew% gegeben. Dazu ist jedoch sorgfältig auf die Abwesenheit von Chlor in den Rohstoffen zu achten. Aus diesem Grunde ist ein Methacrylsäureanhydrid, hergestellt nach dem in USP 5,491,244 beschriebenen Verfahren aus Benzoylchlorid, weniger geeignet. Besser geeignet als Ausgangsmaterial ist ein Methacrylsäureanhydrid, das aus Methacrylsäure und Acetanhydrid hergestellt worden ist. Ein derartiges Produkt ist beispielsweise in DE-OS 3510035 beschrieben. Üblicherweise enthält Methacrylsäureanhydrid, das aus Methacrylsäure durch Umsetzung mit Acetanhydrid hergestellt worden ist, geringe Anteile an nicht umgesetztem Acetanhydrid sowie das gemischte Anhydrid aus Methacrylsäure und Essigsäure. Im allgemeinen ist bei einem chlorfrei hergestellten Methacrylsäureanhydrid eine Reinheit von 93% oder besser 96% ausreichend. Bedingt durch den Gehalt an gemischtem Anhydrid und an Acetanhydrid im Methacrylsäureanhydrid erhält man bei der Umsetzung mit dem Hydroxyacrylat (II) den entsprechenden Essigsäureester (IV) als Verunreinigung.

(IV) CH₂=CHCOO-[-(CH₂)ₓ-CHR-O-]ₙ-COCH₃

Es hat sich jedoch gezeigt, dass im Unterschied zu chlorhaltigen Nebenprodukten eine Verunreinigung von < 5 Gew% (IV), bevorzugt < 2Gew% (IV) und ganz besonders bevorzugt < 1 Gew% (IV) tolerierbar ist. Die Herstellung von unsymmetrischen (Meth)acrylsäurevernetzern (I) mit einem Gehalt an 0,2- 5Gew% (IV) ist daher ein bevorzugtes Verfahren. Dies ermöglicht den Einsatz von technisch gut zugänglichem Methacrylsäureanhydrid als Ausgangsmaterial.
Die Hydroxyacrylate (II) sind im Markt verfügbar. Zu Polyoxyalkylenen siehe z.B. Ullmann's Encyclopedia of Industrial Chemistry,Vol.A21, 579.
Hydroxyethylacrylat bzw. Hydroxypropylacrylat werden z.B. von Gruber et al. beschrieben (USP 3,987,090). 4- Hydroxybutylacrylat ist gemäss EPA 0465853 zugänglich.
Besonderes Interesse finden unsymmetrische (Meth)acrylatvernetzer (I) auf Basis einer Polyoxyethylengruppe. Dies entspricht x= 1, R= H in Formel (I). Diese Verbindungen sind erfindungsgemäss durch Umsetzung von Polyethylenglykol- acrylat (= Polyethylenoxid- acrylat) x= 1, R= H in Formel (II) mit Methacrylsäureanhydrid zugänglich.
Als Ausgangsmaterial zu nennen sind Verbindungen mit n= 1 - 100. Interesse finden insbesondere Diethylenglykolacrylat (n=2), Triethylenglykolacrylat (n=3), Tetraethylenglykolacrylat (n=4) und Mischungen mit n= 2- 40, insbesondere n= 4- 20 und ganz besonders bevorzugt mit einem Mittelwert für n im Bereich 5- 10. Als Beispiel ist hier Polyethylenglykol- acrylat mit einem mittleren Molekulargewicht von Mₙ ca. 375 zu nennen.
Ebenso von Interesse sind unsymmetrische (Meth)acrylatvernetzer (I) auf Basis einer Polypropylenoxydkette. Dies entspricht x=1, R= CH₃ in Formel (I). Als Ausgangsmaterial dienen die entsprechenden Acrylsäureester gemäss Formel (II) im Bereich n= 1-100. Besonderes Interesse findet Hydroxypropylacrylat (n= 1) sowie die entsprechenden Oligomeren n=2-40. Von besonderem Interesse sind auch hier Mischungen mit n= 4-20 bzw. einem mittleren n im Bereich 5-10. Beispielhaft ist ein Polypropylenglykol- acrylat mit einem mittleren Molekulargewicht von Mₙ ca. 475 zu nennen.

Interessant sind darüber hinaus (Meth)acrylatvernetzer, die sowohl Oxyethyl- als auch Oxypropyl- Einheiten enthalten.
Ebenso zu nennen sind unsymmetrische (Meth)acrylatvernetzer (I) auf Basis einer Polytetrahydrofurankette (x= 3, R= H in Formel (I)). Von besonderem Interesse sind hier 4- Hydroxybutylacrylat (n= 1) sowie Mischungen mit n= 2- 50.
Als saure Katalysatoren verwendet man vorzugsweise Schwefelsäure, aromatische oder aliphatische Sulfonsäuren, die z.B. an ein Polymerharz gebunden sein können, oder Phosphonsäuren. Diese Katalysatorsäuren werden zweckmässigerweise in Anteilen von 0,3- 5 Gew% bezogen auf das eingesetzte Methacrylsäureanhydrid eingesetzt. Ein geringerer Anteil an Katalysatorsäure ist ungeeignet, da dies zu langen Reaktionszeiten und Nebenreaktionen führt.
Im allgemeinen wird die Reaktion innerhalb von 2 bis 12h durchgeführt.Die Reaktionstemperatur liegt im allgemeinen im Bereich 30-80°C.
Wesentlich für das Gelingen der Umsetzung der Hydroxyacrylate (II) zu den Produkten (I) ist eine ausreichende Stabilisierung des Ansatzes gegen Polymerisation. Hier sind insbesondere sterisch gehinderte Phenole wie Topanol A oder Ionol zu nennen. Sofern die Hydroxyacrylate (II) weitere Stabilisatoren wie beispielsweise Hydrochinonmonomethylether enthalten, so werden diese im allgemeinen zu den entsprechenden Methacrylsäurephenylestern umgesetzt. Hydrochinmonomethylether ist daher als alleiniger Stabilisator ungeeigenet. Im Gegenteil, es empfiehlt sich, für die erfindungsgemässe Herstellung von unsymmetrischen (Meth)acrylatvernetzern bereits mit sterisch gehinderten Phenolen stabilisiertes Hydroxyacrylate (II) einzusetzen.

Im allgemeinen wird das molare Verhältnis von Methacrylsäureanhydrid zu Hydroxyacrylat (II) im Bereich 1:1 gewählt. Insbesondere bei längerkettigen Molekülen (n=2-10), wird mit Vorteil ein Unterschuss an Methacrylsäureanhydrid (z.B. 0,95 Teile Methacrylsäureanhydrid / 1,0 Teile Hydroxyacrylat) eingesetzt. Dies gewährleistet einen vollständigen Umsatz des Methacrylsäureanhydrids, so dass eine Abtrennung von nicht umgesetztem Anhydrid entfällt.
Ebenso ist es möglich, einen Überschuss an Methacrylsäureanhydrid, von z.B. 20 Mol % zu verwenden. In diesem Fall wird nach möglichst vollständiger Umsetzung des Hydroxyacrylates (II) das überschüssige Methacrylsäureanhydrid durch Zusatz eines niedermolekularen Alkohols wie Methanol, Ethanol oder Isopropanol zerstört. Dieses Verfahren empfiehlt sich insbesondere bei längerkettigen Verbindungen (n=3- 100).
Dabei garantiert der Überschuss an Methacrylsäureanhydrid eine möglichst vollständige Umsetzung der Hydroxyverbindung, die nachfolgende Umsetzung des Methacrylsäureanhydrids zu beispielsweise Ethylmethacrylat und Methacrylsäure gewährleistet eine leichtere Abtrennung dieser im Vergleich zu Methacrylsäureanhydrid niedriger siedenden Komponenten.
Prinzipiell kann die bei der Umsetzung des Hydroxyesters mit Methacrylsäureanhydrid gebildete Methacrylsäure direkt aus dem Reaktionsansatz destilliert werden. Daneben ist es auch möglich, die Umsetzung erst vollständig durchzuführen, den Katalysator abzutrennen und dann erst die Methacrylsäure aus dem Ansatz zu destillieren. Auf jeden Fall erfolgt die Abtrennung der Methacrylsäure bei reduziertem Druck, z.B. bei p< 10mbar. Dabei wird zur besseren Stabilisierung Luft durch den Reaktionsansatz geleitet.
Im allgemeinen wird die Methacrylsäure bis auf einen Gehalt von < 5 Gew%, bevorzugt < 2Gew% aus dem Ansatz entfernt.
Bei den kurzkettigen unsymmetrischen (Meth)acrylatvernetzern (n=1) ist eine Reinigung der Produkte (I) durch nachfolgende Destillation bei reduziertem Druck möglich, bevorzugt ist jedoch die Herstellung der Verbingungen (I) ohne eine Reinigung durch Destillation.
Dazu wird z.B. der Katalysator durch Filtration oder Zentrifugation abgetrennt, im Falle nicht polymergebundener Katalysatoren wie z.B. Schwefelsäure oder Methansulfonsäure kann auch ein Entfernen des Katalysators durch Waschen mit Wasser durchgeführt werden. Die Abtrennung der Methacrylsäure erfolgt- wie oben dargestellt- durch Destillation.
Das auf diesem Wege anfallende Produkt ist im allgemeinen so rein, Gehalt an (Meth)acrylatvernetzer (I) > 90, bevorzugt > 95, besonders bevorzugt > 98%, dass das Produkt ohne weitere Reinigung für die meisten Anwendungen eingesetzt werden kann.
Hier ist insbesondere die Verwendung der (Meth)acrylatvernetzer (I) zum Aufbau zweiphasiger Polymersysteme, z.B. PMMA- Polybutylacrylat-Mischpolymerer, wie sie z.B. als schlagzäh modifiziertes PMMA von Interesse sind.
Von besonderer Bedeutung sind jedoch (Meth)acrylatvernetzer (I), die einen Restgehalt an 1- 40 Gew%, bevorzugt 3- 20 Gew% an Hydroxyacrylaten (II) enthalten. Derartige Vernetzer sind besonders einfach bei Einsatz eines entsprechenden Unterschusses an Methacrylsäureanhydrid herstellbar. Diese unsymmetrischen Vernetzer sind bevorzugt zur Herstellung von sehr homogenen, sehr weichen, hydrophilen Netzwerken geeignet. Hier ist insbesondere die Herstellung von Superabsorbern zu nennen.
Besonderes Interesse finden die mittels des erfindungsgemässen Verfahrens hergestellten Vernetzer (I) auch zur Herstellung von pHabhängigen Verdickungsmitteln, z.B. zur Synthese von Emulsionspolymerisaten auf Basis Ethylacrylat/ Methacrylsäure/Vernetzer, wie sie z.B. in wässerigen Lackrezepturen eingesetzt werden.
In diesem Fall ist eine Abtrennung der Methacrylsäure nicht erforderlich.
In einer einfachen Eintopfreaktion werden das Hydroxyacrylat (II) und Methacrylsäureanhydrid mit Hilfe eines Katalysators zu einer Mischung aus unsymmetrischem (Meth)acrylatvernetzer und Methacrylsäure umgesetzt.
Die dabei anfallende Mischung kann direkt oder nach Abtrennung oder Neutralisation der Katalysatorsäure zur Herstellung von (meth)acrylsäurehaltigen Netzwerken eingesetzt werden.
So enthalten die Rezepturen für derartige alkalisch wirksame Verdickungsmittel im allgemeinen 10- 50 Gew% Methacrylsäure und 0,01- 5Gew% an Vernetzer. Dies allein zeigt, dass eine Abtrennung der Methacrylsäure für derartige Anwendungen nicht erforderlich ist. Die besonders einfache Herstellung einer Mischung aus unsymmetrischen (Meth)acrylatvernetzern (I) und Methacrylsäure ist daher ein weiteres Ziel dieser Erfindung.

## Patentansprüche

1. Verfahren zur Herstellung von unsymmetrischen (Meth)acrylatvernetzern der Formel (I)
(I) CH₂=CHCOO-[-(CH₂)ₓ-CHR-O-]ₙ-COCCH₃=CH₂
mit x = 1, 2, 3, R = H, CH₃, n = 1- 100
**dadurch gekennzeichnet, dass** Acrylsäureester vom Typ (II)
(II) CH₂=CHCOO-[-(CH₂)ₓ-CHR-O-]ₙ-H
mit Methacrylsäureanhydrid unter Bildung von (I) und Methacrylsäure umgesetzt werden.

2. Verwendung der nach dem Verfahren gemäß Anspruch 1 hergestellten unsymmetrischen (Meth)acrylatvernetzern zur Herstellung von Superabsorbern.

3. Verwendung der nach dem Verfahren gemäß Anspruch 1 hergestellten unsymmetrischen (Meth)acrylatvernetzern zur Herstellung von Verdickungsmitteln.

## Claims

1. Process for preparing asymmetric (meth)acrylate crosslinking agents of formula (I)
(I) CH₂=CHCOO-[-(CH₂)ₓ-CHR-O-]ₙ-COCCH₃=CH₂
where x = 1, 2, 3, R = H, CH₃, n = 1-100
**characterised in that** acrylic acid esters of type (II)
(II) CH₂=CHCOO-[-(CH₂)ₓ-CHR-O-]ₙ-H
are reacted with methacrylic anhydride, forming (I) and methacrylic acid.

2. Use of the asymmetric (meth)acrylate crosslinking agents prepared by the process according to claim 1, for the production of superabsorbers.

3. Use of the asymmetric (meth)acrylate crosslinking agents prepared by the process according to claim 1, for the production of thickeners.

## Revendications

1. Procédé de préparation d'agents réticulants de type (méth)acrylate asymétriques de formule (I)
(I) CH₂=CHCOO-[-(CH₂)ₓ-CHR-O-]ₙ-COCCH₃=CH₂
dans laquelle x = 1, 2, 3, R = H, CH₃, n = 1 - 100 **caractérisé en ce qu'**
on fait réagir des esters d'acide acrylique de type (II)
(II) CH₂=CHCOO-[-(CH₂)ₓ-CHR-O-]ₙ-H
avec de l'anhydride de l'acide méthacrylique avec formation de (I) et d'acide méthacrylique.

2. Utilisation des agents réticulants de type (méth)acrylate asymétriques selon la revendication 1, pour la production de superabsorbants.

3. Utilisation des agents réticulants de type (méth)acrylate asymétriques selon la revendication 1, pour la production d'agents épaississants.
